# EUROPÄISCHE PATENTSCHRIFT

(11) **EP 2 283 143 B1**
(45) Veröffentlichungstag und Bekanntmachung des Hinweises auf die Patenterteilung: **27.12.2017**
(21) Anmeldenummer: 09761585.0
(22) Anmeldetag: 22.05.2009
(51) Int. Cl.: C12P 13/02, C07H 15/00, A61K 8/68, A61Q 19/00

(54) **VERFAHREN ZUR HERSTELLUNG VON SPHINGOLIPIDEN DURCH N-ACYLIERUNG VON LYSOSPHINGOLIPIDEN UNTER VERWENDUNG EINER FUNGALEN LIPASE**
PROCESS FOR PRODUCING SPHINGOLIPIDS BY N-ACYLATION OF LYSOSPHINGOLIPIDS EMPLOYING A FUNGAL LIPASE
PROCÉDÉ DE PRODUCTION DE SPHINGOLIPIDES PAR N-ACYLATION DE LYSOSPHINGOLIPIDS EN UTILISANT UNE LIPASE FONGIQUE

(30) Priorität: 13.06.2008 DE 102008002409
(43) Veröffentlichungstag der Anmeldung: 16.02.2011
(73) Patentinhaber: Evonik Degussa GmbH, 45128 Essen (DE)
(72) Erfinder: HOLLMANN, Frank, NL-2522 JJ Den Haag (NL); THUM, Oliver, 40880 Ratingen (DE); TÖLLE, Christoph, 47167 Duisburg (DE); PROVINZANO, Angelo, 46236 Bottrop (DE); KOREVAAR, Cornelis Gerrit Nijs, NL-2341 KB Oegstgeest (NL)
(86) Internationale Anmeldenummer: PCT/EP2009/056206
(87) Internationale Veröffentlichungsnummer: WO 2009/150022

(56) Entgegenhaltungen:
- WO-A-94/26919
- WO-A-97/09307
- WO-A-2006/002909
- TAKANAMI, T. ET AL.: "Chemo-enzymatic short-step total synthesis of symbioramide" TETRAHEDRON LETTERS, Bd. 46, Nr. 19, 9. Mai 2005 (2005-05-09), Seiten 3291-3295, XP025385668

## Beschreibung

### Gebiet der Erfindung

Gegenstand der Erfindung ist die enzymatische Synthese von Sphingolipiden aus Lysosphingolipiden und Carbonsäureestern.

### Stand der Technik

Die Erfindung betrifft ein Verfahren zur biokatalytischen Herstellung von Sphingolipiden der allgemeinen Formel I 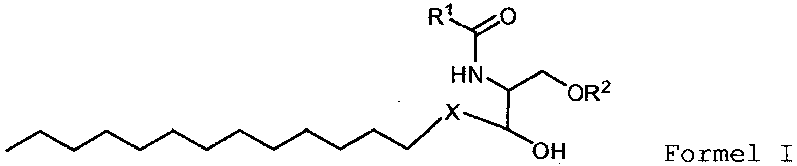 durch Herstellung eines Lysosphingolipids der allgemeinen Formel II 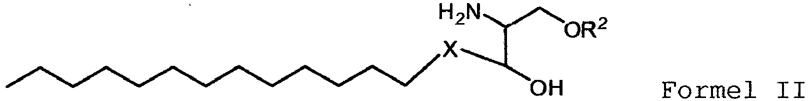 durch Deprotonierung eines Säureadditionsprodukts,
und Umsetzung des Lysosphingolipids mit einem Carbonsäureester der allgemeinen Formel III 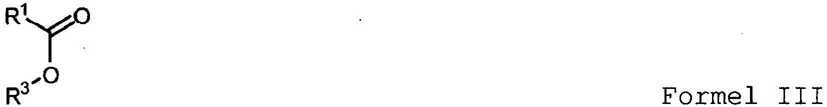
wobei R¹ eine lineare oder verzweigte, gegebenenfalls eine oder mehrere Mehrfachbindungen und/oder aromatische oder heteroaromatische Ringe enthaltende, gegebenenfalls durch Sauerstoffatome oder Ester- oder Amidfunktionalitäten unterbrochene, gegebenenfalls durch mindestens eine weitere Gruppe ausgewählt aus Alkyl-Hydroxy-, Keto- oder Amingruppen substituierte Alkylkette mit 2 bis 55 Kohlenstoffatomen darstellt,
R² H, Phosphocholin, Ethanolamin, Serin oder einen Zucker darstellt,
X CH=CH, CH₂ -CH₂ oder CH₂-HCOH darstellt,
R³ einen verzweigten oder unverzweigten Alkylrest mit 1 bis 12 C-Atomen, der mit mindestens einem Rest -OR⁴ substituiert sein kann, darstellt,
wobei R⁴ unabhängig voneinander gleiche oder ungleiche Reste ausgewählt aus der Gruppe umfassend H und -C(O)R¹ mit R¹ wie oben definiert ist,
dadurch gekennzeichnet, dass ein Biokatalysator eingesetzt wird, der mindestens eine Carboxylester-Hydrolase der Enzymklasse E.C. 3.1.1 ausgewählt aus der Gruppe umfassend
Carboxylester-Hydrolasen der Enzymklasse E.C. 3.1.1, die sich aus einem Organismus des Reiches der Pilze isolieren lassen,
und Carboxylester-Hydrolasen der Enzymklasse E.C. 3.1.1, die auf Aminosäureebene mindestens 80 % homolog zu den sich aus einem Organismus des Reiches der Pilze isolieren lassenden sind,
aufweist,
und dass zur Deprotonierung eine organische oder anorganische Base eingesetzt wird, die nach Protonierung kein Wasser frei setzt.

Die Sphingolipide finden Anwendung als kosmetisch und/oder dermatologisch aktive Komponenten.

Sphingolipide der allgemeinen Formel I mit R² = H werden Ceramide genannt und sind natürlich in der Haut (Stratum corneum) vorkommende polare Lipide. In den äußeren Hornzellen (Corneocyten) stellen Ceramide einen Hauptbestandteil (40-65% des Lipidanteils) der Zellmembranen dar und spielen somit eine zentrale Rolle in deren Schutzfunktion indem sie beispielsweise die Wasserpermeabilität regulieren. Mit zunehmendem Alter büßen die Keratinozyten einen Großteil ihrer Ceramidsynthese-aktivität ein wodurch die Haut einen Teil ihrer Schutzwirkung verliert und beispielsweise den epidermalen Wasserverlust nicht mehr vollständig unterbinden kann. Dies kann durch topische Applikation von Ceramiden wenigstens teilweise kompensiert werden (z.B.: Farwick, M. et al., Int. J. Cosm. Sci., 2007, 29(4), 319-329 oder Klee, S.K. et al. Basic Clinic. Dermatol., 2007, 40, 155-165).

Darüber hinaus wurden positive Effekte von Ceramiden bei der Behandlung atopischer Dermatitis berichtet (Kerscher, M. et al., Eur. J. Dermatol., 1991, 1, 39-43; Imokawa, G. et al., J. Soc. Cosmet. Chem. 1989, 40, 500-507).

In Anbetracht der demographischen Entwicklung in vielen Industrieländern, insbesondere in Deutschland, ist mit einem weiter wachsenden Bedarf an Ceramiden zu rechnen.

Nach dem Stand der Technik werden Ceramide durch Schotten-Baumann-analoge N-Acylierung von Lysosphingolipiden der allgemeinen Formel II mit R² = H wie z.B. Phytosphingosin (allgemeine Formel II mit R² = H und X = CH₂-HCOH) mittels aktivierter Carbonsäurederivate hergestellt.

Typischerweise ist das Phytosphingosin fermentativen Ursprungs.

Bei der aktivierten Carbonsäure handelt es sich typischerweise um das Carbonsäurechlorid welches entweder als solches eingesetzt wird oder *in situ* aus der Carbonsäure hergestellt wird. In US6420604 (Cosmoferm, NL) wird die Synthese einiger Ceramide durch Reaktion der Sphingosinbase mit entsprechenden Carbonsäurehalogeniden beschrieben. Besonders nachteilig bei dieser Methode ist einerseits die Notwendigkeit des Einsatzes von toxischen Organochlorverbindungen sowie das Anfallen einer hohen Salzfracht im Endprodukt. Außerdem ist dem Fachmann offensichtlich, dass bei der Verwendung hochreaktiver Carbonsäurehalogenide mit der Bildung signifikante Mengen unerwünschter Nebenprodukte zu rechnen ist.

Alternative Syntheserouten verlaufen über Anhydride. So lehrt beispielsweise WO93/20038 (Gist-Brocades, NL) die basenkatalysierte Synthese gemischter Anhydride aus Carbonsäure und Alkyl-phenylsulfonylchlorid um reaktive Carbonsäurederivate für die N-Acylierung von Phytosphingosin zu erhalten.

Allen diesen Routen ist gemein, dass sie auf der Verwendung reaktiver Carbonsäurederivate beruhen. Nachteilig ist hierbei sowohl die hohe Korrosivität dieser Substanzen als auch ihre Gesuridheitsschädlichkeit was spezielle Reaktorsysteme und Vorkehrungen notwendig macht und somit den präparativen Aufwand erhöht. Darüber hinaus muss für die topische Anwendbarkeit der Produkte sichergestellt sein dass sie von den reaktiven und toxischen Edukten und Nebenprodukten befreit sind. Des Weiteren ist mit hohen Salzfrachten zu rechnen sowie mit dem damit verbundenen zusätzlichen Aufwand bei der Produktaufreinigung und Entsorgung. Ein weiterer Nachteil der Verfahren des Standes der Technik ist, dass nur geringe Edukt- und Produktkonzentrationen erhalten werden; so sind in WO93/20038 (Gist-Brocades, NL) maximal 15% (w/v) -Eduktlösungen im toxischen Lösungsmittel Methylenchlorid bei gleichzeitiger Verwendung von reaktiven und toxischen Coreagentien p-Toluolsulfonsäurechlorid und Triethylamin beschrieben. Darüber hinaus sind entweder Reinheit der Produkte oder Ausbeuten (jeweils im Bereich um 80%) nicht zufriedenstellend.

Aufgabe der vorliegenden Erfindung war deshalb die Bereitstellung eines alternativen, schonenden und industriell anwendbaren Zugangs zu Ceramiden bei dem wenigstens einer der Nachteile des Standes der Technik überwunden werden kann.

Amidierungsreaktionen sind auch biokatalytisch, d.h. unter Verwendung eines Enzyms als Katalysator, zugänglich. Übersichten über industrielle Anwendungen von Enzymen finden sich beispielsweise bei Liese et al (Industrial Biotransformations; Second, Completely Revised Edition, Wiley-VCH, Weinheim, 2006). Bevorzugte Biokatalysatoren für die Synthese von Carbonsäurederivaten sind Hydrolasen (E.C. 3.1.x.x) insbesondere Lipasen (Triacylglyceridesterhydrolasen, E.C. 3.1.1.3) und Esterasen (E.C. 3.1.1.1).

Entsprechend ihrer natürlichen Funktion im Metabolismus einer Zelle katalysieren Lipasen bevorzugt hydrolytische Spaltung von Estern. Aber auch die kondesative Bildung von Estern ist vielfach literaturbeschrieben. Repräsentative Beispiele hierfür finden sich bei Drauz und Waldmann (Enzyme Catalysis in Organic Synthesis, A Comprehensive Handbook; Second, Completely Revised and Enlaged Edition, Vol. II, Wiley-VCH, Weinheim, 2002), bei Aehle (Enzymes in Industry, Production and Applications; Second, Completely Revised Edition, Wiley-VCH, Weinheim, 2004) oder bei Bornscheuer (Enzymes in Lipid Modification, Wiley-VCH, Weinheim, 2000).

Auch für die Verwendung von Aminen als Nucleophile in Lipase-katalysierten Kondensationsreaktionen finden sich Literaturbeispiele.

Y.-M. Xia et al, J Mol Catal B, 2004, 31,111-115 beschreibt beispielsweise die Synthese von N-Lauroyl-β-aminoproprionitril durch Amidierung von Laurinsäuremethylester mit einem reaktiven, primären Amin katalysiert durch eine Lipase aus *Candida antarctica* (CALB). Nachteilig bei dem beschriebenen Verfahren ist neben der Beschränkung auf verdünnte Substratlösungen (unter 50 mM) der vergleichweise geringe Umsatz von lediglich bis zu 94,3% Umsatz. Darüber hinaus ist bei der gezeigten Reaktion keinerlei Regio- oder Chemoselektivität notwendig.

Solche Selektivitäten können allerdings bei der erfindungsgemäßen Umsetzung zwingend notwendig, da sich in einem Eduktmolekül wie beispielsweise dem Phytosphingosin mehrere reaktive Funktionalitäten befinden können. Die Problematik dieser Selektivität zeigt sich beispielsweise bei P. Tufvesson et al.: Biotechnol.Bioeng., 2007, 97(3), 447-453: Bei der CALB-katalysierten Umsetzung von Ethanolamin mit Carbonsäuren war keine einfache selektive Amidierung unter Umgehung der enzymkatalysierten Veresterung möglich. Lediglich unter mehrfachem Zudosieren eines Eduktes und destillativer Entfernung des Reaktionswassers konnte eine Anreicherung des gewünschten Amids erreicht werden.

In einem weiteren Beispiel für die enzymatische Amidierung beschreiben M. Nechab et al.: JOC, 2007, 72, 6918-6923 die stereoselektive Umsetzung von (R)-konfigurierten sekundären Aminen mittels CALB. Die hohe optische Reinheit (>99% ee) der beobachteten Produkte legt eine starke Präferenz der CALB für (R)-konfigurierte Amine nahe, wodurch die Umsetzung von beispielsweise Phytosphingosin aufgrund seiner S-Konfiguration am Amin-Kohlenstoffatom mit diesem Katalysator fraglich ist. Des Weiteren wurden wiederum stark verdünnte Substratlösungen (100 mM) bei gleichzeitig hohen Biokatalysatorkonzentrationen (27% w(CALB)/w(Substrate)) eingesetzt was die industrielle Attraktivität des beschriebenen Verfahrens signifikant einschränkt.

Das Dokument TAKANAMI, T. ET AL.: "Chemo-enzymatic short-step total synthesis of symbioramide": TETRAHEDRON LETTERS, 2005, 46(19), 3291-3295 offenbart ein Verfahren zur biokatalytischen Herstellung eines Sphingolipids der Formel I, nämlich Symbioramid, durch Umsetzung des entsprechenden Lysosphingolipids der Formel II mit einem Carbonsäureester der Formel III in Gegenwart der Lipase Chirazyme L-2 (Lipase B aus *Candida antarctica*), wobei das Lysosphingolipid der Formel II vor der enzymatischen Umsetzung über mehrere chemische Reaktionen aus Methyl-trans-2,3-epoxyoctadecanonat hergestellt wird.

Die Patentschrift WO94/26919 (Gist-Brocades, NL) offenbart ein Verfahren zur biokatalytischen Herstellung eines Sphingolipids der Formel I durch Umsetzung des entsprechenden Lysosphingolipids der Formel II mit einem Carbonsäureester der Formel III in Gegenwart der *Pseudomonas alcaligenes* Lipase M1, wobei vorgeschlagen wird, das Lysosphingolipid der Formel II vor der enzymatischen Umsetzung durch Deprotonierung eines Säureadditionsprodukts des Lysosphingolipids unter Verwendung einer Base, die nach der Protonierung Wasser frei setzt, herzustellen.

In WO94/26919 ist die enzymatische N-Acylierung von Phytosphingosin mittels Carbonsäureestern und unter Verwendung bakterieller Lipasen oder Säugetierlipasen beschrieben. In dem beschriebenen Verfahren werden bevorzugt bakterielle Lipasen und Säugetierlipasen eingesetzt; insbesondere eine Lipase aus *Pseudomonas alcaligenes.* Nachteilig bei diesem Verfahren ist insbesondere die Notwendigkeit hoher Biokatalysatormengen (30-95% w/w) zur Erreichung von nur mäßigen Umsätzen (bis zu 78%). Darüber hinaus wurden signifikante Mengen an unerwünschten N,O-Di-acylierungsprodukten (bis zu 17%) gefunden. Zusätzlich wurden lediglich verdünnte Lösungen der Substrate eingesetzt (59 bis 93 mM) was geringe Raum-Zeit-Ausbeuten zur Folge hatte. Ein weiterer Nachteil ist die Beschränkung auf Phytosphingosin-Base als Substrat was im Vergleich zur Verwendung von Säureadditionsprodukten des Phythosphingosins, beispielsweise Phytosphingosin-Sulfat, eine drastische Erhöhung der Substratkosten mit sich bringt. Ausdrücklich wird außerdem darauf hingewiesen, dass Lipasen aus Hefen und Pilzen als Katalysatoren nicht geeignet sind. Somit scheidet das in WO94/26919 beschriebene Verfahren als ökonomisch sinnvolle Alternative zu den bestehenden chemischen Verfahren aus.

Es besteht daher weiterhin Bedarf an Methoden der enzymatischen Amidierung von Lysosphingolipiden welche die Nachteile des Standes der Technik überwinden, um bisher nicht realisierbare biokatalytische Spingolipidsynthese ausgehend von Carbonsäurealkylestern zu ermöglichen.

### Beschreibung der Erfindung

Aufgabe der vorliegenden Erfindung war daher die Bereitstellung eines Verfahrens, das einen oder mehrere Nachteile der Verfahren des Standes der Technik nicht aufweist. Insbesondere sollte ein Verfahren entwickelt werden, mit dem gut verfügbare Lysosphingolipide als Edukte und darüber hinaus in hohen Konzentrationen eingesetzt werden können.

Weitere nicht explizit genannte Aufgaben ergeben sich aus dem Kontext der nachfolgenden Beschreibung, der Beispiele sowie der Ansprüche.

Überaschenderweise wurde gefunden, dass entgegen dem Stand der Technik mittels Pilzlipasen die selektive Umsetzung von Carbonsäureestern mit Lysosphingolipiden zu Sphingolipiden hoher Reinheit möglich ist. Darüber hinaus wurde gefunden, dass diese Umsetzung ökonomisch sinnvoll durchführbar ist.

Das erfindungsgemäße Verfahren bzw. der erfindungsgemäß verwendete Biokatalysator hat den Vorteil, dass Amidierungsreaktion ökonomisch sinnvoll durchgeführt werden können, da entweder relativ niedrige Enzymkonzentrationen eingesetzt werden müssen, beispielsweise unter 10 Gewichts-% bezogen auf die eingesetzten Edukte, und/oder der Biokatalysator mehrfach, zum Beispiel mindestens 10-fach, wieder verwendet werden kann. Ein weiterer Vorteil des erfindungsgemäßen Verfahrens besteht darin, dass ohne aufwändige Aufarbeitungsreaktionen, wie Chromatographie oder fraltionierte Kristallisation Produkte hoher Reinheit, beispielsweise mit einem Aktivgehalt von > 90% und einem Anteil von N,O-Diacetylierungsprodukten von unter 5% darstellbar sind. Ein besondere Vorteil des erfindungsgemäßen Verfahrens besteht darin, dass als Carbonsäurekomponenten einfach zugängliche Carbonsäurealkylester, beispielsweise Methylester, eingesetzt werden, die keine unerwünschten Nebenreaktionen verursachen.

Gegenstand der vorliegenden Erfindung ist daher ein Verfahren zur biokatalytischen Herstellung von Sphingolipiden der allgemeinen Formel I 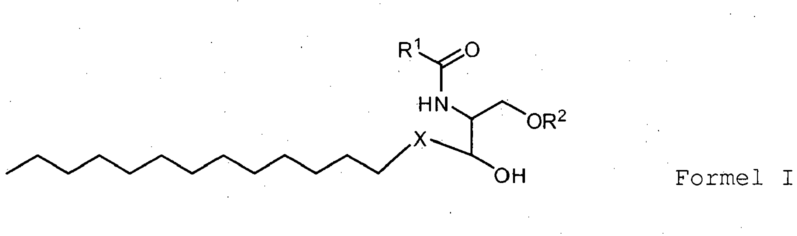 durch Herstellung eines Lysosphingolipids der allgemeinen Formel II 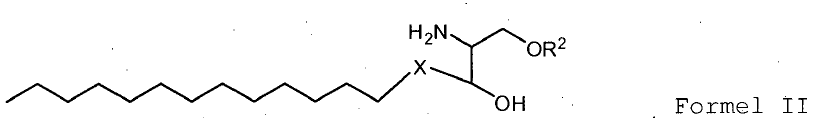 durch Deprotononierung eines Säureadditions produkts, und Umsetzung des Lysosphingolipids mit einem Carbonsäureester der allgemeinen Formel III, 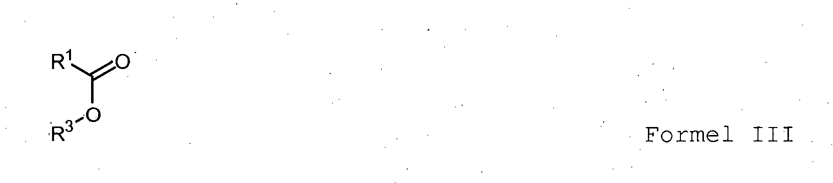
wobei R¹ eine lineare oder verzweigte, gegebenenfalls eine oder mehrere Mehrfachbindungen und/oder aromatische oder heteroaromatische Ringe enthaltende, gegebenenfalls durch Sauerstoffatome oder Ester- oder Amidfunktionalitäten unterbrochene, gegebenenfalls durch mindestens eine weitere Gruppe ausgewählt aus Alkyl- Hydroxy-, Keto- oder Amingruppen substituierte Alkylkette mit 2 bis 55 Kohlenstoffatomen, bevorzugt -CH₂-Y-CH₃ mit Y = eine Kohlenstoff-Kohlenstoff-Bindung oder eine lineare oder verzweigte, gegebenenfalls eine oder mehrere Mehrfachbindungen und/oder aromatische oder heteroaromatische Ringe enthaltende, gegebenenfalls durch Sauerstoffatome oder Ester- oder Amidfunktionalitäten unterbrochene, gegebenenfalls durch mindestens eine weitere Gruppe ausgewählt aus Alkyl- Hydroxy-, Keto- oder Amingruppen substituierte Alkylenkette mit 1 bis 53 Kohlenstoffatomen, darstellt,
R² H, Phosphocholin, Serin, Ethanolamin oder einen Zucker bevorzugt Zucker oder H, besonders bevorzugt H darstellt,
X CH=CH, CH₂-CH₂ oder CH₂-HCOH, bevorzugt CH₂-HCOH darstellt,
R³ einen verzweigten oder unverzweigten Alkylrest mit 1 bis 12 C-Atomen, der mit mindestens einem Rest -OR⁴ substituiert sein kann, darstellt,
wobei R⁴ unabhängig voneinander gleiche oder ungleiche Reste ausgewählt aus der Gruppe umfassend
H
   und
   ein Rest -C(O)R¹
ist,
dadurch gekennzeichnet, dass ein Biokatalysator eingesetzt wird, der mindestens eine Carboxylester-Hydrolase der Enzymklasse E.C. 3.1.1 ausgewählt aus der Gruppe umfassend
Carboxylester-Hydrolasen der Enzymklasse E.C. 3.1.1, die sich aus einem Organismus des Reiches der Pilze isolieren lassen
   und
   Carboxylester-Hydrolasen der Enzymklasse E.C. 3.1.1, die auf Aminosäureebene mindestens 60 %, vorzugsweise mindestens 80 % bevorzugt mindestens 90 %, besonders bevorzugt mindestens 95%, 98% bzw. 99 % homolog zu den sich aus einem Organismus des Reiches der Pilze isolieren lassenden sind,
aufweist, und dass zur Deprotonierung eine organische oder anorganische Base eingesetzt wird, die nach Protonierung kein Wasser fole setzt.

Mit "Homologie auf Aminosäureebene" im Sinne der vorliegenden Erfindung ist nun und sei auch im Folgenden die "Aminosäure-Identität" verstanden, welche mit Hilfe bekannter Verfahren bestimmt werden kann. Generell werden besondere Computerprogramme mit Algorithmen unter Berücksichtigung spezieller Erfordernisse verwendet. Bevorzugte Verfahren zur Bestimmung der Identität erzeugen zunächst die größte Übereinstimmung zwischen den zu vergleichenden Sequenzen. Computer-Programme zur Bestimmung der Identität umfassen, sind jedoch nicht beschränkt auf, das GCG- Programmpaket, einschließlich
- GAP (Deveroy, J. et al., Nucleic Acid Research 12 (1984), Seite 387, Genetics Computer Group University of Wisconsin, Medicine (Wi), und
- BLASTP, BLASTN und FASTA (Altschul, S. et al., Journal of Molecular Biology 215 (1990), Seiten 403-410. Das BLAST-Programm kann erhalten werden vom National Center For Biotechnology Information (NCBI) und aus weiteren Quellen (BLAST Handbuch, Altschul S. et al., NCBI NLM NIH Bethesda ND 22894; Altschul S. et al., vorstehend).

Der Fachmann ist sich bewusst, dass verschiedene Computer-programme für die Kalkulation der Ähnlichkeit bzw. Identität zwischen zwei Nukleotid- oder Aminosäure-Sequenzen zur Verfügung stehen. So kann die prozentuale Identität zwischen zwei Aminosäure-Sequenzen z.B. durch den Needleman und Wunsch (J. Mol. Biol. (48): 444-453 (1970)) Algorithmus bestimmt werden, der in das GAP Programm im GCG Software-Paket (erhältlich über http://www.gcg.com) integriert wurde, berechnet werden und zwar entweder unter Verwendung einer Blossom 62-Matrix oder einer PAM250-Matrix, einer gap weight von 16, 14, 12, 10, 8, 6, oder 4 und einer length weight von 1, 2, 3, 4, 5, oder 6. Der Fachmann wird anerkennen, dass die Verwendung unterschiedlicher Parameter zu leicht unterschiedlichen Ergebnissen führen wird, dass aber die prozentuale Identität zwischen zwei Aminosäure-Sequenzen insgesamt nicht signifikant unterschiedlich sein wird. Üblicherweise wird die Blossom 62-Matrix unter Anwendung der Voreinstellungen (gap weight: 12, length weight: 1) genutzt.

Eine Identität von 60 % gemäß dem vorstehenden Algorithmus bedeutet im Zusammenhang mit der vorliegenden Erfindung 60 % Homologie. Das gleiche gilt für höhere Identitäten.

Besonders bevorzugt ist in dem erfindungsgemäßen Verfahren der Carbonsäureester der allgemeinen Formel III der Ester einer Carbonsäure mit einem Rest -R³ des Alkohols R³OH, bei dem die Carbonsäure ausgewählt ist aus der Gruppe der natürlich vorkommenden Fettsäure auf Basis natürlicher pflanzlicher oder tierischer Öle mit 6-30 Kohlenstoffatomen, insbesondere mit 8-22 Kohlenstoffatomen. Natürliche Fettsäuren sind unverzweigt und bestehen aus einer geraden Anzahl Kohlenstoffatomen. Eventuelle Doppelbindungen besitzen cis-Konfiguration. Beispiele sind: Capronsäure, Caprylsäure, Caprinsäure, Laurinsäure, Myristinsäure, Palmitinsäure, Palmitoleinsäure, Isostearinsäure, Stearinsäure, 12-Hydroxystearinsäure, Dihydroxystearinsäure, Ölsäure, Linolsäure, Linolensäure, Petrolesinsäure, Elaidinsäure, Arachinsäure, Behensäure, Erucasäure, Gadoleinsäure, Linolensäure, Eicosapentaensäure, Docosahexaensäure, Arachidonsäure, dessen Esterprodukte ganz besonders bevorzugt sind.

Des Weiteren ist in dem erfindungsgemäßen Verfahren der Carbonsäureester der allgemeinen Formel III bevorzugt der Ester einer Carbonsäure mit einem Rest -R³ des Alkohols R³OH, bei dem die Carbonsäure ausgewählt ist aus der Gruppe der hydroxylierten Derivaten einer mehrfach ungesättigten Fettsäure. Solche Fettsäure sind beispielsweise 9- oder 13-Hydroxyoctadecadiensäure, 15-Hydroxyeicosatetraensäure und die Reihe der sogenannten alpha-Hydroxysäuren. Ebenfalls ist in diesem Zusammenhang die Carbonsäure besonders bevorzugt ein Polykondensationsprodukt von hydroxyfunktionalisierten Säuren, beispielsweise Poly-12-hydroxystearinsäure oder Polyricinolsäure.

Ebenfalls ist in diesem Zusammenhang die Carbonsäure besonders bevorzugt eine synthetische oder natürlich vorkommende Carbonsäure, die aromatische Substituenten enthält, wie z.B. Benzoesäure, Zimmtsäure, Ferulasäure, Protocatechursäure, Gallussäure, Vanillinsäure, Syringasäure, Isoferulasäure, Sinapinsäure, Kaffesäure, Genitinsäure, Salicylsäure, Salicylursäure oder Nikotinsäure.

In dem erfindungsgemäßen Verfahren ist R³ bevorzugt ein unsubstituierter Alkylrest mit 1 bis 4 C-Atomen, bevorzugt ist R³ ausgewählt aus der Gruppe enthaltend Methyl-, Ethyl-, Vinyl-, Propyl-, Isopropyl-, n-Butyl, sek-Butyl- und tert-Butyl-Reste.

In einer weiteren Ausführungsform des erfindungsgemäßen Verfahrens stellt der Carbonsäureester der allgemeinen Formel III einen Voll- oder Partialestern von Polyolen mit mindestens einer Säure R¹COOH dar. Bevorzugt werden als Voll- oder Partialester von Polyolen Glycol-, Glyceryl-, 1,2-pentandiyl- 1,3-pentandiyl-, 1,2-butandiyl-, 1,3-Butadiyl-, 1,4-Butadiylester, sowie deren Isomere und ungesättigte Analoga eingesetzt.

Die durch die Veresterung von R¹COOH den Rest R³ bestimmenden oben genannten Edukte können als Reinsubstanz oder im Gemisch vorliegen, so dass folglich durch die Struktur der allgemeinen Formel III gegebenenfalls ein Gemisch beschrieben werden kann.

In dem erfindungsgemäßen Verfahren können Mischungen von Sphingolipiden der allgemeinen Formel I biokatalytisch hergestellt werden, indem Mischungen der allgemeinen Formel III mit unterschiedlichem R¹ eingesetzt werden.
Ebenso können in dem erfindungsgemäßen Verfahren Mischungen von Carbonsäureestern der allgemeinen Formel III mit unterschiedlichem R³ eingesetzt werden.

Erfindungsgemäß werden die Säureadditionsprodukte, auch Säureadditionsalze genannt, der Lysosphingolipide eingesetzt, wie sie beispielsweise im Falle des bereits genannten Phytosphingosins bei den etablierten fermentativen Verfahren anfallen, wie beispielsweise beschrieben bei P. Lersch, U. Schick, Spec. Chem. Mag., 2003, 23(6), 30-31. Als Säureadditionsprodukt des Lysosphingolipids wird bevorzugt das Carbonsäurecarboxylat, Sulfat, Phosphat, Nitrat, Carbonat, Hydroxid oder Halogenid, besonderd bevorzugt das Chlorid und Sulfat des Lysosphingolipids eingesetzt.

Bei Verwendung des Säureadditionsproduktes des Lysosphingolipids in erfindungsgemäßem Verfahren wird das Lysosphingolipid durch Deprotonierung des Säureadditionsprodukts des Lysosphingolipids vor der enzymatischen Umsetzung hergestellt.

Das so erhaltene Lysosphingolipid wird im Folgenden auch als Lysosphingolipid-Base bezeichnet.

Die deprotonierende Behandlung kann in Lösungen oder Suspensionen des Säureadditionsproduktes des Lysosphingolipids in üblichen organischen Lösungsmitteln stattfinden. Als Lösungsmittel können beispielsweise eingesetzt werden: Paraffine, ein- oder mehrwertige Alkohole (z.B.: Methanol, Ethanol, Isopropanol, Propanol, Butanol, Pentanol, Hexanol, Cyclohexanol, Methylcyclohexanol, 2-Butyl-1-octanol sowie deren Isomere, Ethylenglycol, Glycerol, Diacetonalkohol, Isobutanol, etc.), Ether (Diethylether, tert.-Butylmethylether, Tetrahydrofuran, Dioxan, Polyethoylate, Polypropoxylate sowie Mischpolymere etc.), Ketone (Azeton, Methylisobutylketon, Methylethylketon, etc.), Ester (Triethylcitrat, Tributylcitrat. Isobutylisobutyrat, Isobutylacetat. Isononylisononanoat,(2-Ethylhexyl)Acetat, Cyclohexylacetat, 4-tert.-Butylcyclohexylacetat). Bevorzugt sind toxikologisch unbedenkliche Lösungsmittel wie Methylisobutylketon oder 2-Methyl-2-butanol.

Der Deprotonierungsschritt kann bei Reaktionstemperaturen durchgeführt werden, bei denen das Lösungsmittel sich im flüssigen Zustand befindet. Vorzugsweise bei Reaktionstemperaturen zwischen -80°C und +150°C, besonders bevorzugt zwischen 0°C und 120°C, ganz besonders bevorzugt zwischen +25°C und 100°C.

Die so erhaltene Lösung von Lysosphingolipid-Base kann als solche für die enzymatische Umsetzung eingesetzt werden oder vorher filtriert werden.

In einer weiteren Ausführung der vorliegenden Erfindung kann das Lösungsmittel des Deprotonierungsschritts nach einer dem Fachmann geläufigen Methode (z.B. destillative Entfernung oder Präzipitation/Kristallisation der Lysosphingolipid-Base mit anschließender Filtration) entfernt werden. Optional kann die Lysosphingolipid-Base vor der enzymatischen Umsetzung beispielsweise durch Präzipitation oder Kristallisation oder durch destillative Entfernung des Lösungsmittels isoliert werden. Bevorzugt ist, dass zwischen der Deprotonierung des Säureadditionsprodukts des Lysosphingolipids und biokatalytischer Herstellung ein Filtrationsschritt erfolgt, in dem die bei der deprotonierenden Behandlung anfallenden Salze entfernt werden.

Die mit einer Aktivierung einhergehende Deprotonierung erfolgt durch Einsatz einer organischen oder anorganischen Base. Bevorzugt werden als anorganische Basen Carbonate, Metallhydride (wie z.B.: Lithiumaluminiumhydrid, Calziumhydrid, Natriumhydrid und ähnliche), Ionenaustauschermaterialien (wie z.B. Kationen- oder Anionentauscher) sowie als organische Basen Metallorganyle (wie beispielsweise Butyllithium) Alkoholate, Amine sowie deren Metallsalze wie beispielsweise Lithiumdiisopropylamin eingesetzt.

Überraschenderweise wurde gefunden, dass solche Basen, bei deren Reaktion mit den Säureadditionssalzen formal kein Wasser entsteht, qualitative besonders hochwertige Lysoshingolipid-Base liefern, welche sich in der enzymatischen Reaktion besonders leicht umsetzen lässt. Es handelt sich also um solche Basen, bei denen kein Reaktionswasser frei wird. Dies sind solche, die nach Protonierung kein Wasser frei setzen. Bevorzugt eingesetzt wird als Base ein Alkalimetallalkoholat. Dieses kann beispielsweise in alkoholischer Lösung vorliegen. Besonders bevorzugt sind Natrium- und Kaliummethanolat. Diese können ebenfalls als Lösungen in organischen Lösungsmitteln eingesetzt werden.

Eine weitere Alternative, Reaktionswasser zu vermeiden, ist der Einsatz von wasserbindenden, bevorzugt wasserbindenden anorganische Salzen, wie beispielsweise Na₂SO₄ zur Bindung von entstehendem Reaktionswasser bei Einsatz von Alkali- und/oder Erdalkalihydroxiden als Base. Bevorzugt wird Na₂SO₄ eingesetzt.

Erfindungsgemäß liegt in dem Verfahren das molare Verhältnis zwischen Säureadditionsprodukts des Lysosphingolipids und Base im Bereich zwischen 10 zu 1 bis 0,05 zu 1, bevorzugt zwischen 3 zu 1 bis 0,2 zu 1, besonders bevorzugt zwischen 1,4 zu 1 und 0,6 zu 1 und ganz besonders bevorzugt ist das molare Verhältnis zwischen Säureadditionsprodukt des Lysosphingolipids und Base äquimolar.

In einer besonderen Ausführung der vorliegenden Erfindung wird eventuell vorhandenes Wasser aus dem Deprotonierungsansätz zur Herstellung der Lysosphingolipid-Base entfernt. Beispielsweise werden hierzu Wasserentfernungsverfähren wie physikalischer Methoden (z.B. Destillation, Membranverfahren, Extraktion oder Adsorption (beispielweise an Molekularsieb)) oder auch chemische Methoden (z.B.: Reaktion mit Metallhydriden und -Oxiden oder anderen Reaktanden wie Anhydriden, Estern) eingesetzt.

Wie bereits eingangs erwähnt, ist es vor dem Stand der Technik, wie er in WO94/26919 (Gist-Brocades) beschrieben ist, völlig überraschend, dass Pilzlipasen in erfindungsgemäßem Verfahren als effizienter Biokatalysator eingesetzt werden können.

In dem erfindungsgemäßen Verfahren werden als Biokatalysator Carboxylester-Hydrolasen der Enzymklasse E.C. 3.1.1, die sich aus einem Organismus des Reiches der Pilze isolieren lassen und/oder die auf Aminosäureebene mindestens 80 % bevorzugt mindestens 90 %, besonders bevorzugt mindestens 95%, 98% bzw. 99 % homolog zu den sich aus einem Organismus des Reiches der Pilze isolieren lassenden sind, bei denen der Organismus ausgewählt ist aus der Gruppe der Gattungen *Aspergillus, Bipolaris, Candida, Fusarium, Geotrichum, Humicola, Microsporum, Mucor, Pichia, Thermomyces, Penicilium, Pichia, Rhizopus, Rhizomucor, Saccharomyces, Thermomyces, Trichosporon, Zygosaccharomyces.*

Beispielhafte Vertreter sind z.B.: *Aspergillus caesiellus, A. candidus, A. carbonarius, A. carneus, A. chevalieri, A. clavatus, A. costaricaensis, A. cretensis, A. deflectus, A. flaviceps, A. flavus, A. flocculatosus, A. fumigatus, A. glaucus, A. ibericus, A. lacticoffeatus, A. lentulus, A. neobridgeri, A. nidulans, A. niger, A. niveus, A. ochraceus, A. oryzae, A. parasiticus*, *A. penicilloides, A. piperis, A. pseudoelegans, A. pseudofisheri, A. restrictus, A. roseoglobulus, A. sclerotioniger, A. sojae, A. steynii, A. sydowi, A. terreus, A. udagawae, A. ustus, A. versicolor, A. westerdijkiae, Bipolaris australiensis, B.hawaiiensis, B. picifera, Candida antarctica, C. cylindracea, C. lipolytica, C. rugosa, C. utilis, C. robusta, C. kefyr, C. albicans, C. glabrata, C. krusei, C. lusitaniae, C. parapsilosis, C. tropicalis, Fusarium chlamydosporum, F. coeruleum, F. dimerum, F. incarnatum, F. moniliforme F. oxysporum, F. proliferatum, F. sacchari, F. semitectum, F. solani, F. sporotrichoides, F. sub glutinans, F. tabacinum, F. verticillioides, Geotrichum candidum, G.klebahnii, Humicola fuscoatra var. Fuscoatra, H.a grisea var. Grisea, H. grisea var. Thermoidea, H. insolens, Microsporum amazonicum, M. audouinii, M. audouinii var. Langeronii, M. audouinii var. Rivalierii, M. boullardii, M. canis, M. canis var. distortum, M. cookei, M. equinum, M. ferrugineum, M. fulvum, M. gallinae, M. gypseum, M. nanum, M. persicolor, M. praecox, M. racemosum, M. vanbreuseghemii Mucor javanicus, M. pusillus, M. plumbeus, M. racemosus, M. hiemalis, Penicillium aurantiogriseum, P. brevicompactum, P. chrysogenum, P. camemberti, P. digitatum, P. citrinum, P. commune*, *P. corylophilum, P. crustosum, P. cyclopium, P. expansum, P. funiculosum, P. glabrum, P. glaucum, P. griseofulvum, P. italicum, P. marneffei, P. nalgiovense, P. nordicum, P. notatum, P. palitans, P. purpurrescens, P. purpurogenum*, *P. olsonii, P. oryzae, P. roqueforti, P. variabile, P. viridicatum, P. verrucosum, Pichia acaciae, P. amylophilia, P. ciferrii, P. pastoris, P. alni, P. americana, P. amethionina, P. angophorae, P. angusta, P. anomala, P. antillensis, P. barkeri, P. besseyi, P. bimundalis, P. bispora, P. bovis, P. cactophila, P. canadensis, P. capsulata, P. caribaea, P. castillae, P. chambardii, P. delftensis, P. deserticola, P. dryadoides, P. euphorbiae, P. euphorbiiphila, P. fabianii, P. faecalis, P. farinosa, P. fermentans, P. finlandica, P. fluxuum, P. galaeiformis, P. glucozyma, P. guilliermondii, P. hampshirensis, P. haplophila, P. heedii, P. heimii, P. henricii, P. holstii, P. inositovora, P. jadinii, P. japonica, P. kluyveri, P. kodamae, P. lynferdii, P. maganishii, P. media, P. membranifaciens, P. methanolica, P. methylivoria, P. mexicana, P. meyerae, P. minuta, P. mississippiensis, P. nakasei, P. nakazawae, P. norvegensis, P. oezlemii, P. ofunaensis, P. ohmeri, P. onychis, P. opuntiae, P. petersonii, P. philodendri, P. philogaea, P. pijperi, P. pini, P. populi, P. pseudocactophila, P. quercuum, P. rabaulensis, P. rhodanensis, P. salicaria, P. scolyti, P. segobiensis, P. silvicola, P. spartinae, P. stipitis, P. strasburgensis, P. subpelliculosa, P. sydowiorum, P. tannicola, P. thermotolerans, P. toletana, P. trehalophila, P. triangularis, P. veronae, P. wickerhamii, P. xylosa, Thermomyces lanuginosa, Rhizopus arrhizus, R: delemar, R. niveus, R. oryzae, R. azygosporus, R. microsporus*, *R. schipperae, R. stolonifer, Rhizomucor miehei, Saccharomyces barnettii, S. bayanus, S. castellii, S. cerevisiae, S. dairenensis, S. exiguus, S. paradoxus, S. pastorianus, S. rosinii, S. servazii, S. spencoerorum, S. transvaalensis, S. unisporus, S. bailii, Trichosporon asahii, T. asteroides*, *T. beigelii, T. coremiiforme, T. cutaneum, T. faecale, T. gracile, T. inkin, T. mucoides, T. ovoides, T. pullulans, Zygosaccharomyces bisporus, Z. cidri, Z. fermantati, Z. florentinus, Z. mellis, Z. microellipsoides, Z. mrakii, Z. rouxii.*

Besonders bevorzugt eingesetzte Carboxylester-Hydrolasen in erfindungsgemäßem Verfahren sind Enzyme ausgewählt aus der Gruppe der Lipase aus *Thermomyces lanuginosus* (accessionnumber *059952),* die Lipasen A und B *(*accessionnumber P41365) aus *Candida antarctica* und, die Lipase aus *Mucor miehei (*accessionnumber *P19515),* die Lipase aus *Rhizomucor javanicus (*accessionnumber S32492), die Lipase aus *Rhizopus oryzae* (accessionnumber P61872), die Lipasen aus *Candida rugosa* (accessionnumber P20261, P32946, P32947, P3294 und P32949), die Lipase aus *Rhizopus niveus (*accessionnumber *P61871),* die Lipase aus *Penicillium camberti (*accessionnumber P25234), die Lipasen aus *Aspergillus niger* (ABG73613, ABG73614 und ABG37906) und die Lipase aus *Penicillium cyclopium* (accessionnumber P61869), so wie jeweils deren auf Aminosäureebene mindestens 80 % bevorzugt mindestens 90 %, besonders bevorzugt mindestens 95%, 98% bzw. 99 % homologen. Bezüglich Homologie sei auf oben genannte Definition verwiesen.

Kommerzielle Beispiele und ebenfalls bevorzugt eingesetzte Carboxylester-Hydrolasen in erfindungsgemäßem Verfahren sind die Handelsprodukte Lipozyme TL IM, Novozym 435, Lipozyme IM 20, Lipase SP382, Lipase SP525, Lipase SP523, (alles Handelsprodukte der Firma Novozymes A/S, Bagsvaerd, Dänemark), Chirazyme L2, Chirazyme L5, Chirazyme L8, Chirazyme L9 (alles Handelprodukte der Firma Roche Molecular Biochemicals, Mannheim, Deutschland), sowie Lipase M "Amano", Lipase F-AP 15 "Amano", Lipase AY "Amano", Lipase N "Amano", Lipase R "Amano", Lipase A "Amano", Lipase D "Amano", Lipase G "Amano" (alles Handelsprodukte der Firma Amano, Japan).

Der Biokatalysator wird vorzugsweise in wasserfreier, bzw. teilhydratisierter Form eingesetzt. Er kann immobilisiert oder als Lyophilisat eingesetzt werden. Als Träger zur Immobilisierung können inerte organische oder anorganische Träger verwendet werden. Vorzugsweise werden als inerte Träger solche partikulären Träger verwendet bzw. sind im Enzymimmobilisat vorhanden, die eine Teilchengrößenverteilung aufweisen, bei der mindestens 90% der Teilchen eine Teilchengröße von 10 bis 5000 µm, bevorzugt von 50 µm bis 2000 µm aufweisen. Als organische Träger können insbesondere solche eingesetzt werden, die Polyacrylat, Polymethacrylat, Polyvinylstyrol, Styrol-Divinylbenzolcopolymeren, Polypropylen, Polyethylen, Polyethylenperepthalat, PTFE und/oder andere Polymere aufweisen oder aus diesen bestehen. Als Trägermaterial können, in Abhängigkeit von dem zu immobilisierenden Enzym, insbesondere saure oder basische Ionenaustauscherharze eingesetzt werden, beispielsweise Duolite A568, Duolite XAD 761, Duolite XAD 1180, Duolite XAD 7HP, Amberlite IR 120, Amberlite IR 400, Amberlite CG 50, Amberlyst 15 (alles Produkte der Fa. Rohm und Haas) oder Lewatit CNP 105 und Lewatit VP OC 1600 (Produkte der Fa. Lanxess, Leverkusen, Deutschland). Als anorganische Träger können aus dem Stand der Technik bekannte, oxidische und/oder keramische Träger eingesetzt werden. Insbesondere können als anorganische Träger beispielsweise Celite, Zeoliten, Silica, Controlled-Pore Glas (CPG) oder andere Träger, wie sie zum Beispiel in L. Cao, "Carrier-bound Immobilized Enzymes: Principles, Application and Design", Wiley-VCH: 2005, Weinheim, Deutschland, beschrieben sind, eingesetzt werden. Besonders bevorzugt bestehen die im Enzymimmobilisat vorhandenen inerten Träger bzw. die zur Herstellung der Enzymimmobilisate verwendeten inerten Träger aus Polyvinylstyrol, Polymethacrylat oder Polyacrylat.

Die Immobilisierung auf den Partikeln kann kovalent oder nichtkovalent, bevorzugt nichtkovalent erfolgen. Für die nichtkovalente Immobilisierung kann der Träger z. B. mit einer wässrigen Enzymlösung, die gegebenenfalls weitere Bestandteile, wie z. B. anorganische Salze oder Detergenzien, enthalten kann, inkubiert bzw. imprägniert werden. Diese Inkubation/Imprägnierung kann z. B. bei Temperaturen zwischen 0 °C und 50 °C, bevorzugt zwischen 0 °C und 40 °C durchgeführt werden. Vorzugsweise erfolgt die Inkubation/Imprägnierung über einen Zeitraum von wenigen Minuten bis zu einigen Stunden. Der Fortschritt der Inkubation kann durch Bestimmung der Konzentration des Enzyms in der Lösung mit den gängigen Verfahren zur Proteinbestimmung erfolgen. Nach Erreichen des gewünschten Immobilisierungsgrads kann der Träger vorzugsweise mit Wasser gewaschen und, wenn gewünscht, getrocknet werden.

Möglich ist auch die Verwendung ganzer Zellen, die einen geeigneten Biokatalysator enthalten, entweder als *resting cells* oder in getrockneter Form, vorzugsweise nach einer Methode des Standes der Technik permeabilisiert.

Erfindungsgemäß kann der Biokatalysator in einem Mengenverhältnis zu den eingesetzten Substraten von 0,01% (w/w) bis 300% (w/w)verwendet werden. Das bevorzugte Massenverhältnis folgt aus der spezifischen Aktivität des Biokatalysators unter den jeweiligen Reaktionsbedingungen (insbesondere in Abhängigkeit der Substrate, deren Konzentration, des Lösungsmittels und der Reaktionstemperatur). In Abhängigkeit dieser Parameter wird eine Biokatalysatormenge bevorzugt, die einen Vollumsatz der Reaktanden innerhalb eines Zeitraumes von 1 bis 96 Stunden, vorzugsweise von 8 bis 24 Stunden ermöglicht. Bevorzugt wird die Biokatalysatormenge bezogen auf die Gesamtmasse an Edukten in einem Bereich zwischen 1% (w/w) und 100% (w/w) und ganz besonders bevorzugt zwischen 1% (w/w) und 50% (w/w) insbesondere zwischen 1% (w/w) und 20% (w/w) eingesetzt.

In einer besonderen Ausführungsform wird der Biokatalysator zurückgewonnen.

Erfindungsgemäß können die Reaktanden zu Beginn der enzymatischen Umsetzung in einem molaren Verhältnis von Lysosphingolipid zu Carbonsäureester von 1:10 bis 10:1 vorliegen. Der Term "molare Verhältnis" bezieht sich hierbei auf das molare Verhältnis von Lysosphingolipid-Aminen zu Acyldonorcarboxylatgruppen. Bevorzugt werden Mengenverhältnisse zwischen 1:3 und 3:1 eingesetzt. Besonders bevorzugt werden Mengenverhältnisse zwischen 1:1,4 und 1,4:1 eingesetzt, insbesondere äquimolare Mengenverhältnisse.

Die Reaktanden können entweder im geschmolzenen Zustand ohne weitere Lösungsvermittler vorliegen oder in einem organischen Lösungsmittel gelöst oder suspendiert sein. Bevorzugt wird die Reaktion im organischen Lösungsmittel durchgeführt. Als Lösungsmittel eignen sich besonders diese die die Reaktanden bei Temperaturen im Bereich 20°C bis 130°C in hohen Konzentrationen zu lösen vermögen. Als besonders geeignet erwiesen sich beispielsweise, ohne eine Beschränkung auf diese Lösungsmittel, Ketone wie beispielsweise Methylisobutylketon oder Cylclohexanon, sterisch gehinderte sekundäre Alkohole wie 2-Butyl-1-Oktanol, Methylcyclohexanole, 1-Methoxy-2-propanol, 2,3-Butandiol 2-Oktanol, Diacetonalkohol, 2-Methyl-2-butanol, sterisch gehinderte Ester wie 4-tert-Butylcyclohexylacetat, (2-Ethylhexyl)acetat, Cyclohexylacetat und Ether wie 1,4-Dioxan, Tetrahydrofuran und Varonic APM (Evonik Goldschmidt GmbH, Essen, Deutschland). Als ungeeignet erwiesen sich solche Lösungsmittel, insbesondere Ester, die mit signifikanter Aktivität vom Biokatalysator umgesetzt werden. Bei der Reaktion mit Phytosphingosin als Edukt und Verwendung von Buttersäureethylester konnten beispielsweise neben dem gewünschten Produkt signifikante Mengen an N-Butyrolyphytosphingosin und Umesterungsprodukt mit dem eingesetzten Acyldonor (z.B. Methylstearat) nachgewiesen werden. Dasselbe gilt für die Verwendung von Essigsäurebutylester (Bildung von N-Acetylphytosphingosin und Acyldonorbutylester).

Bevorzugt wird die Reaktion unter wasserfreien Bedingungen, definiert als Wassergehalt von maximal 0,100 M, bevorzugt maximal 0,010 M und besonders bevorzugt unter maximal 0,005 M, nachgewiesen nach Karl Fischer, durchgeführt.

Hohe Substratkonzentrationen sind besonders vorteilhaft für die Reaktionsgeschwindigkeit. Bei hohen Umsätzen resultieren daraus hohe Produktkonzentrationen die sich überraschenderweise aber nicht negativ auf die Enzymaktivität bei hohen Umsätzen auswirken. Dies ist für den Fachmann insofern überraschend als dass Produktinhibition bei enzymatischen Reaktionen üblicherweise beobachtet wird. Darüber hinaus wurde überraschenderweise gefunden, dass die Entfernung eines der Reaktionsprodukte (Ceramid oder Alkohol) aus der Reaktionsmischung nicht zwingend zur Erreichung sehr hoher bis quantitativer Umsätze notwendig ist. Nach dem, dem Fachmann geläufigen, Prinzip von Le Chatelier wäre dies bei der vorliegenden Gleichgewichtsreaktion zu erwarten gewesen. Darüber ließen die Reaktionsumsätze des Standes der Technik (WO94/26919, Gist-Brocades) von lediglich 67% bis 78% diese Notwendigkeit erwarten. Erfindungsgemäß können allerdings auch niedrige Substratkonzentrationen effizient umgesetzt werden.

Bevorzugt im Sinne der Erfindung ist eine Reaktandenkonzentration zu Beginn der Reaktion im Bereich zwischen 0,01 M und 3 M, insbesondere im Bereich zwischen 0,2 M und 2 M und besonders bevorzugt im Bereich zwischen 0,5 M und 1,5 M je Reaktand liegt.

Bezüglich der Reaktionstemperatur wurde gefunden, dass insbesondere solche Temperaturen geeignet sind bei denen die Substrate homogen im Lösungsmittel löslich sind. Vorteilhaft erwiesen sich dabei Reaktionstemperaturen im Bereich zwischen 20°C und 130°C. Insbesondere bei hohen Temperaturen ist aufgrund von Farbveränderungen auf eine sorgfältige Entgasung der Reaktionsmischung zu achten. Überaschenderweise wurde allerdings gefunden, dass Vorkehrungen solcherart bei Reaktionstemperaturen von 100°C und darunter nicht notwendig sind. Bevorzugt sind daher Reaktionstemperaturen im Bereich von 40 °C bis 90°C und ganz besonders bevorzugt der Bereich zwischen 70 °C und 85 °C.

Die Reaktion wird bevorzugt unter einem Druck von kleiner 1 bar, bevorzugt kleiner 0,5 bar und besonders bevorzugt kleiner 0,05 bar durchgeführt.

Die Reaktionsführung kann im batch-Verfahren (Rührkessel) oder im Festbettreäktor kontinuierlich oder semi-kontinuierlich durchgeführt werden. Insbesondere beim batch-Verfahren war von einer hohen Inaktivierungsrate des Biokatalysators auszugehen. Zu Nennen ist hier die mechanische Zerstörung des Biokatalysators aufgrund des auftretenden mechanischen Stresses durch Rühren, sowie thermische Inaktivierung des Biokatalysators gepaart mit inaktivierenden Effekten hervorgerufen durch Reaktanden und Lösungsmittel. Solche Effekte sind dem Fachmann bekannt und adäquate Abhilfen sind literaturbekannt. So kann beispielsweise bei auftretender mechanischer Abnutzung des Biokatalysators im Rührverfahren diese durch Einsatz eines Festbetts umgangen werden. Weitere Reaktortypen wie sie dem Fachmann bekannt sind eignen sich ebenfalls zur Verhinderung von mechanischer Enzyminaktivierung.

Weiterhin ist bei der Verwendung von Säureestern als Acyldonoren mit chemischer Inaktivierung des Biokatalysators durch die freiwerdenden Alkohole zu rechnen (z.B.: Y. Yoshida et al., J. Biosci. Bioeng., 2006, 102(1), 66-68). Methoden zur Umgehung solcher Inaktivierungen sind dem Fachmann bekannt und umfassen beispielsweise die destillative Abtrennung des Alkohols aus dem Reaktionsgemisch oder die Anwendung von Membranverfahren. Überraschenderweise wurde allerdings gefunden, dass auch ohne Anwendung der oben beschriebenen Verfahren der Biokatalysator mehrfach und ohne signifikanten Aktivitätsverlust wiederverwendet werden kann.

Allen im Stand der Technik beschriebenen Verfahren ist gemein, dass die erhaltenen Produkte Zusammensetzungen aus Sphingolipid und hohen Anteilen (bis zu 19% des Gesamtproduktes) des korrespondierenden N,O-Diacylierungsproduktes sind. Diese Zusammensetzungen sind allesamt in beispielsweise kosmetischen Komposititionen nicht einsetzbar, da der Anteil an N,O-Diacylierungsprodukt nicht kleiner 5 % ist. Überaschenderweise wurde weiterhin gefunden, dass in dem erfindungsgemäßen Verfahren die Amidierung der sekundären Aminfunktion gegenüber der Veresterung einer der Alkoholfunktionen deutlich bevorzugt ist. Ein gegenteiliges Verhalten insbesondere eine Bevorzugung der primären, also sterisch leicht zugänglichen Alkoholfunktion wäre zu erwarten gewesen. Ähnliches ist im Stand der Technik (WO94/26919, Gist-Brocades) beschrieben; die dort erhaltenen Reaktionsprodukte enthielten bei Verwendung einer bakteriellen Lipase und bereits bei einem molaren Überschuss von 60% des Acyldonors über Phytosphingosin, signifikante Anteile an N,O-Diacylierungsprodukt (Mono- zu Diacylierungsprodukt > 1:5).

In demerfindungsgemäßen Verfahren konnte hingegen gezeigt werden, dass sogar bei einem 3,6-fachen Überschuss an Acyldonor über Lysosphingolipid keine signifikante Diacylierung eintritt solange noch Lysosphingolipid im Reaktionsansatz vorhanden ist. Erst ab vollständigem Umsatz von Lysosphingolipid zum Sphingolipid konnte langsame Veresterungsaktivität nachgewiesen werden. Bei stöchiometrischer Verwendung von Lysosphingolipid und Acyldonor wurde die oben beschriebene unerwünschte Nebenreaktion kaum beobachtet.

Offenbart wird ein Verfahren mit dem besonders reine N-Acylphytosphingosine bereits als Rohprodukte aus der enzymatischen Umsetzung erhalten werden. Vorzugsweise liegt der Umsatz in Bezug auf die Zielverbindung gemäß Formel I bei über 80 %, besonders bevorzugt bei über 85% und ganz besonders bevorzugt bei mehr als 90%% des theoretisch zu erwartenden Umsatzes.

Das beschriebene Verfahren eignet sich weiterhin zur Herstellung einer Zusammensetzung enthaltend Formel I und von 0,001 bis 19 Massen -%, bevorzugt 0,005 bis 19 Massen-% besonders bevorzugt 0,01 bis 5 Massen-% des korrespondierenden N,O-Diacetlyierungsproduktes.

Somit sind Zusammensetzungen enthaltend Formel I und von 0,001 bis 19 Massen-%, bevorzugt 0,005 bis 19 Massen-% besonders bevorzugt 0,01 bis 5 Massen-% des korrespondierenden N,O-Diacetlyierungsproduktes, ebenfalls offenbart.

Weiterhin offenbart ist eine Methode zur Isolierung des Sphingolipids aus der Reaktionsmischung. Aufgrund seiner vorteilhaften physikalischen Eigenschaften kann das Sphingolipid leicht durch Kristallisation / Präzipitation aus der Reaktionslösung entfernt werden und somit die bereits hohe Reinheit des Rohproduktes auf einfache Weise noch gesteigert werden.

Beschriebene Sphingolipide und Zusammensetzungen sind besonders geeignet für die Herstellung von kosmetischen und pharmazeutischen Formulierungen. Daher sind kosmetische oder pharmazeutische Formulierungen enthaltend mindestens ein erfindungsgemäßes Sphingolipid und/oder eine beschriebene Zusammensetzung ebenfalls offenbart.

In den nachfolgend aufgeführten Beispielen wird die vorliegende Erfindung beispielhaft beschrieben.

Folgende Abbildungen sind Bestandteil der Beispiele:
Abbildung 1: Reaktionsverlauf einer enzymatischen Umsetzung von Phytosphingosi mit einem 3,4-fachen Überschuss an Acyldonor.
Abbildung 2: Vergleich der Reaktionsverläufe der enzymatischen Umsetzung von Phytosphingosine mit Methylstearat. Dreiecke: mit zusätzlichem N-Stearoylphytosphingosin, Quadrate: ohne zusätzliches N-Stearoylphytosphingosin.
Abbildung 3: Recycling des Biokatakysators: widergegeben sind in schwarz die Anfangsaktivitäten der betreffenden Cyclen und in schraffiert die Ausbeuten nach 24h.
Abbildung 4: Zeit-Umsatz-Kurven der enzymatischen Umsetzung von Phytosphingosin mit Methylstearat in verschiedenen Lösungsmitteln.
Abbildung 5: Verlauf einer enzymatischen CeramidIII-Synthese unter Verwendung von Tristearin als Acyldonor.

### Beispiele:

### Beispiel 1: Deprotonierung von PS-Sulfat (nicht erfindungsgemäß)

100 g Phytosphingosin-Sulfat (Cosmoferm, Delft, NL) werden in 600 g Methanol bei 55°C suspendiert, mit 46 g Natriummethanolat versetzt und 150 Minuten bei 55°C gerührt. Anschließend wird die Suspension heiß filtriert und das Filtrat eingeengt. Der hell-beige Feststoff (78g) wird für weitere Versuche eingesetzt

### Beispiel 2: Screening kommerzieller Lipasen auf Amidierungsreäktivität (nicht erfindungsgemäß)

In einer äquimolaren Lösung von Phytosphingosin (aus Beispiel 1) und Methylstearat (jeweils 1M) in Dioxan (11 mL) werden je 0,35 g (5% w/w) der in Tabelle 1 aufgeführten Pilzlipasen suspendiert und 72h bei 80°C Lipase geschüttelt. Anschließend wird der Biokatalysator heiß abfiltriert und das Filtrat gaschromatographisch analysiert.

**Tabelle 1: Untersuchung verschiedener Hefe- oder Pilzlipasen bezüglich ihrer Eignung als N-Acylierungskatalysator für Phytosphingosin.**

| **Lipase aus** | **% Ceramid** | **% Phytosphingosin** | **% Methylstearat** |
|---|---|---|---|
| *Rhizomucor miehei* | 15,0 | 10,9 | 6,8 |
| *Thermomyces lanuginosa* | 7,1 | 17,3 | 12,9 |
| *Candida rugosa* | 3,6 | 16,8 | 17,7 |
| *Candida cylindracea* | 3,2 | 14,6 | 15,7 |
| *Candida antarctica* (Lipase A) | 2,9 | 20,2 | 15,6 |
| *Candida antarctica* (Lipase B) | 23,1 | 5,3 | 4,4 |

Für weitere Versuche wurde die Lipase B aus *Candida antarctica* verwendet.

### Beispiel 4: Negativkontrolle (nicht erfindungsgemäß)

Um eine mögliche unkatalysierte Umsetzung von Phytosphingosin mit dem Acyldonor auszuschließen wurde Phytosphingosin 0,2 M in Methylstearat bei 80°C gelöst und 4 Stunden inkubiert. Die anschließende gaschromatographische Analyse zeigte keinen signifikanten (quantifizierbaren) Umsatz.

### Beispiel 5: Reaktionen mit Phytosphingosin-n-Sulfat (nicht erfindungsgemäß)

Zur Demonstration der Notwendigkeit der Deprotonierung von Phytosphingosin-Sulfat wurde folgendes Vergleichsexperiment durchgeführt: 8,18g Phytosphingosin-Sulfat und 6,72g Methylstearat wurden in 15 mL Dioxan bei 80°C suspendiert und mit 0,16g Novozym 435 versetzt. Nach 4 Stunden Inkubation unter Rühren wurde die Reaktionsmischung gaschromatographisch analysiert. Es wurden lediglich 31,7 mM N-Stearoylphytosphingosin gefunden. In einem Vergleichsexperiment unter identischen Bedingungen aber nach Zugabe von 2,4g Natriumcarbonat wurden 368 mM N-Stearoylphytosphingosin gefunden.

### Beispiel 6: Regioselektivität der enzymatischen Acylierung (nicht erfindungsgemäß)

Um einen hohen Überschuss von Acyldonor zu Phytosphingosi zu erreichen, wurden 4,43g Phytosphingosin in 15,09g Methylstearat bei 120°C gelöst. Zu dieser Lösung wurden 0,98g Novozym 435 (5% w/w) zugegeben und unter Normaldruck gerührt. Periodisch wurden Proben entnommen und gaschromatographisch analysiert.

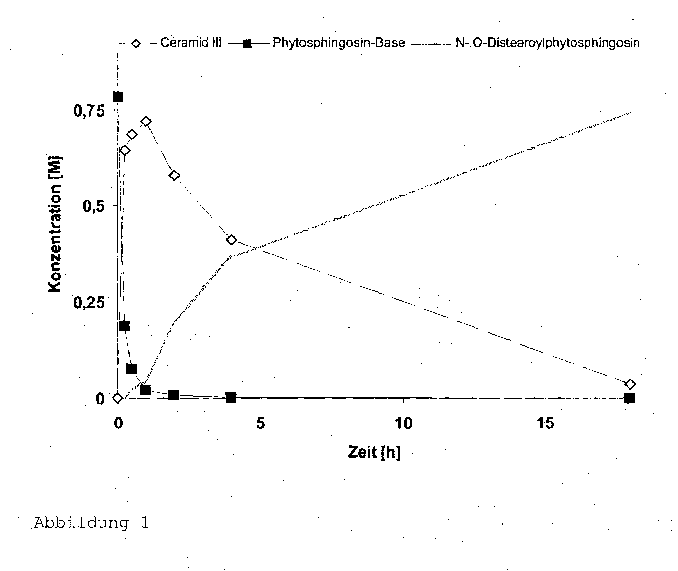

Wie aus dem Reaktionsverlauf (Abbildung 1) erkennbar ist, erfolgt eine signifikante Akkumulation des unerwünschten N,O-Distaerylphytosphingosins erst bei vollständigem Umsatz der eingesetzten Phytosphingosin. So ist nach einer Stunde, bei einer Rest-Phytosphingosinkonzentration von 19 mM (Umsatz > 99,5%) noch kein N,O-Diacylierungsprodukt nachweisbar; erst unterhalb einer Phytosphinigosinkonzentration von ca. 5 mM wird Bildung des N,O-Diacylierungsprodukts beobachtet. Darüber hinaus liegt die Reaktionsgeschwindigkeit der Nebenreaktion um einen Faktor von mehr als 25 unter der der erwünschten Reaktion.

### Beispiel 7: Versuch zur Produktinhibition (nicht erfindungsgemäß)

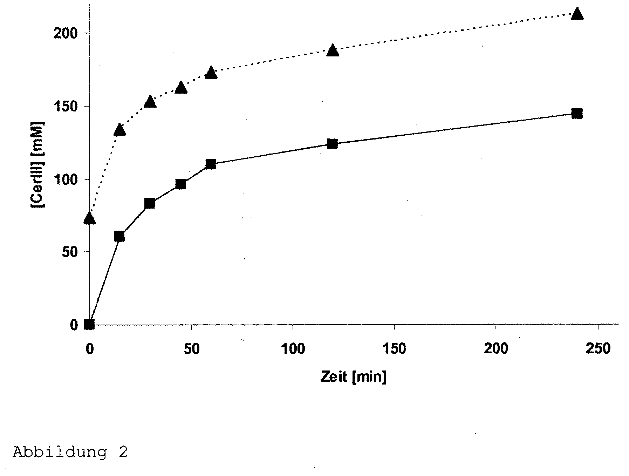

Zum Ausschluss etwaiger Produktinhibition wurden 2 parallele Versuche durchgeführt. Dazu wurden jeweils 1,59g Phytosphingosin zusammen mit 7,45g Methylstearat in 25 mL Dioxan bei 80°C gelöst und mit je 0,49g Novozyme 435 versetzt. Zu einem der beiden Reaktionsmischungen wurde zusätzlich 1,3 g N-Stearoylphytosphingosin (Cosmoferm, Delft, NL) beigemischt. Wie der Vergleich der beiden Zeit-Umsatz-kurven zeigt (Abbildung 2) sind die Reaktionsverläufe identisch so dass eine ausgeprägte Produktinhibition ausgeschlossen werden kann.

### Bespiel 8: Wiederverwertbarkeit des Biokatalysators (nicht erfindungsgemäß)

Zur Evaluation der Wiederverwertbarkeit des Biokatalysators wurde eine Versuchsreihe wie folgt durchgeführt: 3,3 g Phytosphingosin wurden zusammen mit 14,9 g Methylstearat in 50 mL Dioxan bei 80°C gelöst und die Reaktion durch Zugabe von 0,98 g Novo435 gestartet. Es werden periodisch Proben entnommen und gaschromatographisch analysiert und daraus Anfangsgeschwindigkeit und Umsatz errechnet. Nach 24h wird der Reaktionsansatz heiß filtriert und das zurückbleibende Enzym dreimal mit jeweils 50ml heißen (50°C) Dioxan gewaschen. Anschließend wird der so recyclisierte Biokatalysator erneut unter gleichen Bedingungen wie oben beschrieben eingesetzt. Dieses Vorgehen wird insgesamt sechsmal wiederholt.

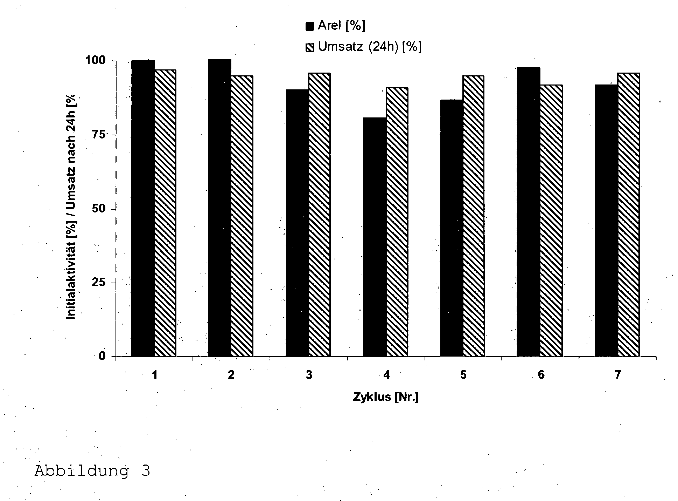

Wie aus Abbildung 3 deutlich wird, kann der Biokatalysator wenigstens siebenmal für die Reaktion eingesetzt werden ohne dass ein Aktivitätsrückgang zu beobachten ist.

### Beispiel 9: Bestimmung der Kinetischen Parameter des Biokatakysators (nicht erfindungsgemäß)

Die kinetischen Parameter der enzymatischen N-Acylierung wurden wie folgt bestimmt: Unter Beibehaltung der Konzentration des einen Reaktanden wurde die Konzentration des anderen Variiert. Die Reaktionen wurden in Dioxan bei 80°C unter Verwendung jeweils der gleichen Enzymmenge durchgeführt. Es wurden periodisch Proben entnommen und gaschromatographisch analysiert; daraus wurden die jeweiligen Anfangsraten bestimmt und diese nach Lineweaver-Burk analysiert. Als Resultat wurden für Phytosphingosin ein K_{M}-Wert von 400 mM und für Methylstearat ein K_{M}-Wert von 196 mM bestimmt.

### Beispiel 10: Einfluss der Temperatur auf die Reaktionsgeschwindigkeit (nicht erfindungsgemäß)

Es wurden enzymatische Umsetzungen bei verschiedenen Temperaturen durchgeführt. Allen gemein war dass die Substrate äquimolar eingesetzt wurden. Die eingesetzte Enzymmenge betrug jeweils 1,4% (w/w). Die Ergebnisse der gaschromatographischen Auswertung der Anfangsgeschwindigkeit sowie des Umsatzes nach 4 Stunden sind in Tabelle 2 widergegeben.

**Tabelle 2: Einfluss der Reaktionstemperatur auf die enzymatische Synthese von N-Stearoylphytosphingosin.**

| T [oC] | Anfangskonzentration [M] / Lösungsmittel | Aspec [Ug-1] | Umsatz nach 4h [%] |
|---|---|---|---|
| 80 | 0,98 (in Dioxan) | 840 | 49,5 |
| 100 | 2,8 (kein) | 1182 | 79 |
| 120 | 2,8 (kein) | 1224 | 92 |

### Beispiel 11: Verwendung verschiedener Lösungsmittel (nicht erfindungsgemäß)

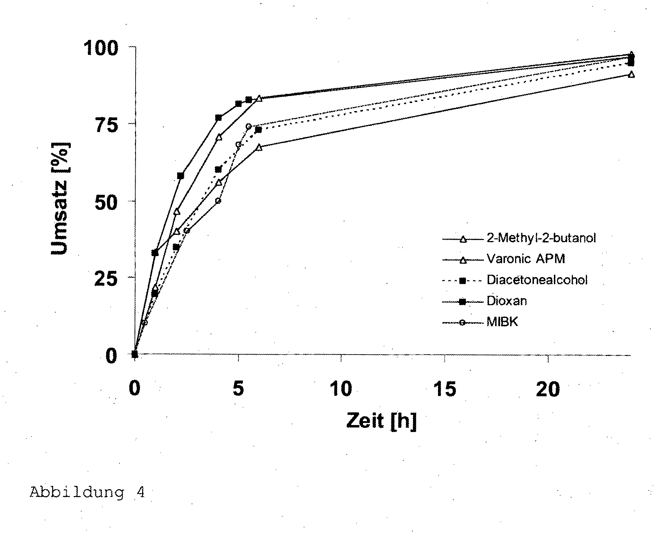

Phytosphingosin und Methylstearat wurden beide 1M in dem jeweiligen Lösungsmittel bei 80°C gelöst, mit 5% (w/w) Novozym 435 versetzt und gerührt. Periodisch wurden Proben entnommen und gaschromatographisch analysiert. Wie aus Abbildung 4 ersichtlich wird, sind die Umsatz-Zeit-Kurven sowie die Umsätze nach 24h der Einzelreaktionen nahezu deckungsgleich.

### Beispiel 12: Enzymatische Umsetzung von Phytosphingosin mit verschiedenen Fettsäureestern (nicht erfindungsgemäß)

Phytosphingosin und die in Tabelle 3 angegebenen Acyldonoren wurden jeweils 1M im Lösungsmittel bei 80°C gelöst, mit 5% (w/w) Novozym 435 versetzt und gerührt. Periodisch wurden Proben entnommen und gaschromatographisch analysiert

**Tabelle 3: Ergebnisse der enzymatischen Umsetzung verschiedener Säureester mit Phytosphingosin.**

| Substrat | Umsatz in % (Reaktionszeit, Lösungsmittel) |
|---|---|
| Hexansäureethylester | 98,2 (24h, MIBK) |
| Ölsäuremethylester | 97,4 (23h, tert-Butanol) |
| Ricinolsäuremethylester | 96,5 (24h, tert-Butanol) |
| o-Salizylsäureethylester | 36,5 (24h, MIBK) |

### Beispiel 13: Selektive Kristallisation des Produkts (nicht erfindungsgemäß)

Methylstearat (734mM) und Phytosphingosin (146 mM) wurden in 2-Methyl-2-butanol bei 80°C gelöst, mit 5% w/w Novozym 435 versetzt und 20h bei 80°C gerührt. Anschließend wurde das Enzym heiß abfiltriert und die klare Ausgangslösung über Nacht bei 45°C temperiert. Der ausgefallene Feststoff wurde abfiltriert, Filtrat und Filterrückstand wurden gaschromatographisch analysiert. Wie aus Tabelle 4 ersichtlich wird ist durch fraktionierte Präzipitation eine selektive Anreicherung des gewünschten Produktes auch in Gegenwart von hohen Substratüberschüssen möglich,

**Tabelle 4: Ergebnisse der selektiven Präzipitation des Reaktionsproduktes aus einer Reaktionsmischung (Angaben: Flächenprozent laut GC).**

| | Methylstearat | Phytosphingosin-Base | CerIII |
|---|---|---|---|
| Ausgangslösung | 69,3 | 0,14 | 30,5 |
| Filtrat | 90,1 | 0,1 | 9,8 |
| Präzipitat | 5,0 | 0,08 | 94,9 |

### Beispiel 14: Tristearin als Acyldonor (nicht erfindungsgemäß)

Es wurden enzymatische Umsetzungen unter Verwendung von Triglycerid (Tristearin) als Acyldonor durchgeführt. Zu einer Lösung von 5,4 g Phytosphingosin und 5g Tristearin in 17g Dioxan bei 80°C wurden 0,5g Novozyme 435 zugegeben und bei Normaldruck gerührt. Es wurden periodisch Proben entnommen und gaschromatographisch analysiert.

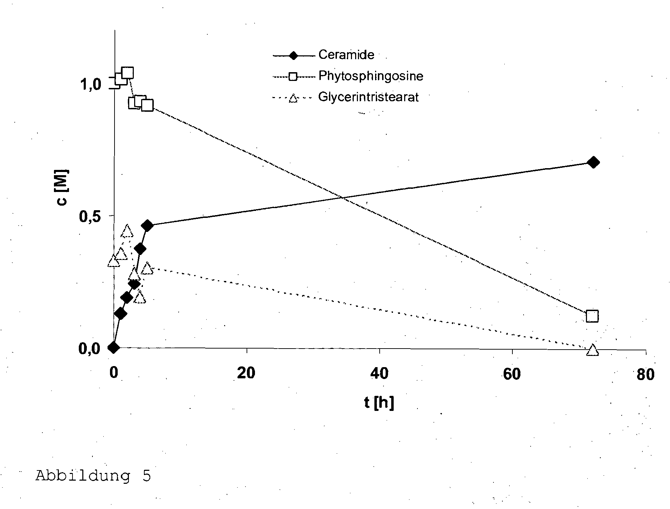

Wie aus Abbildung 5 deutlich wird, ist ein vollständiger Umsatz des Acyldonors möglich. Überraschenderweise ergab die gaschromatographische Analyse dass intermediär keine Partialglyceride (Glycerindistearat oder Glycerinmonostearat) in signifikanten Mengen beobachtbar waren.

### Beispiel 15: Eintopfsynthese von N-Stearoylphytosphingosin ausgehend von Phytosphingosin-Sulfat

100g Phytosphingosin-Sulfat (Cosmoferm, Delft, NL) werden in 100ml MIBK (Methylisobutylketon) bei 55°C suspendiert, mit 46g Natriummethanolat versetzt und 180 Minuten bei 55°C gerührt. Anschließend werden 73,3 g Methylstearat und 5g Novo435 hinzugegeben. Die so erhaltene Suspension wird 36 Stunden bei 80°C gerührt und heiß abfiltriert. Das klare Filtrat wird 4 Stunden bei Raumtemperatur stehen gelassen und der ausgefallene hell-beige Feststoff (122,3g N-Stearoylphytosphingosin, Reinheit >97%) abfiltriert.

### Beispiel 16: Pilotansatz im Fetbettreaktor

207,8 g Phytosphingosin-Sulfat werden zusammen mit 108,04 g NaOCH₃-Lsg (25%ig in Methanol) in 500 ml MIBK bei 55°C suspendiert und anschließend heiß über eine Filterpresse in einen Festbettreaktor überführt und mit 149,3g Methylstearat versetzt. Im Festbett befinden sich 17,8g Novozyme 435. Der gesamte Reaktor ist auf 80°C thermostatisiert. Das Festbett wird mit einer Pumprate von ca. 25 ml min⁻¹ mit der Reaktionsmischung durchspült. Es wird ein Unterdruck von 0,8 bar angelegt, um überschüssiges Methanol zu entfernen. Nach 24h wird der Reaktor mit Stickstoff belüftet, entleert und die Reaktionslösung über Nacht bei Raumtemperatur stehengelassen. Der ausgefallene Feststoff wird abfiltriert (287,1g N-Stearoylphytosphingosin, Reinheit 98,7%).

## Patentansprüche

1. Verfahren zur biokatalytischen Herstellung von Sphingolipiden der allgemeinen Formel I 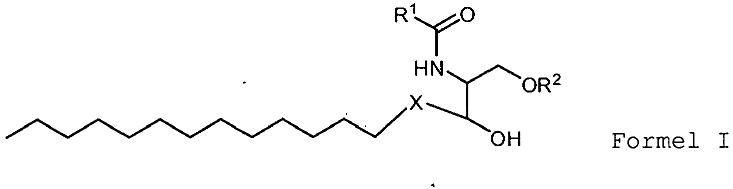 durch
Herstellung eines Lysosphingolipids der allgemeinen Formel II 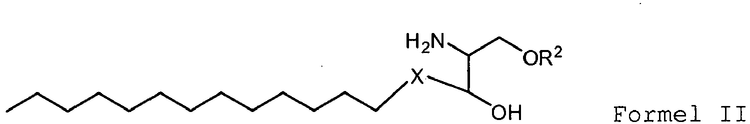 durch Deprotonierung eines Säureadditionsprodukts, und Umsetzung des Lysosphingolipids mit einem Carbonsäureester der allgemeinen Formel III 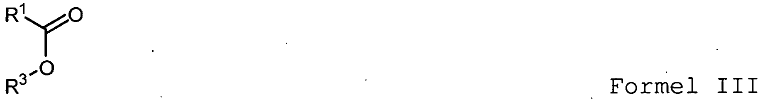
wobei R¹ eine lineare oder verzweigte, gegebenenfalls eine oder mehrere Mehrfachbindungen und/oder aromatische oder heteroaromatische Ringe enthaltende, gegebenenfalls durch Sauerstoffatome oder Ester- oder Amidfunktionalitäten unterbrochene, gegebenenfalls durch mindestens eine weitere Gruppe ausgewählt aus AlkylHydroxy-, Keto- oder Amingruppen substituierte Alkylkette mit 2 bis 55 Kohlenstoffatomen darstellt,
R² H, Phosphocholin, Ethanolamin, Serin oder einen Zucker darstellt,
X CH=CH, CH₂-CH₂ oder CH₂-HCOH darstellt,
R³ einen verzweigten oder unverzweigten Alkylrest mit 1 bis 12 C-Atomen, der mit mindestens einem Rest -OR⁴ substituiert sein kann, darstellt,
wobei R⁴ unabhängig voneinander gleiche oder ungleiche Reste ausgewählt aus der Gruppe umfassend
H
und
-C(O)R¹ mit R¹ wie oben definiert
ist,
**dadurch gekennzeichnet, dass** ein Biokatalysator eingesetzt wird, der mindestens eine Carboxylester-Hydrolase der Enzymklasse E.C. 3.1.1 ausgewählt aus der Gruppe umfassend
Carboxylester-Hydrolasen der Enzymklasse E.C. 3.1.1, die sich aus einem Organismus des Reiches der Pilze isolieren lassen
und
Carboxylester-Hydrolasen der Enzymklasse E.C. 3.1.1, die auf Aminosäureebene mindestens 80 % homolog zu den sich aus einem Organismus des Reiches der Pilze isolieren lassenden sind,
aufweist, und
dass zur Deprotonierung eine organische oder anorganische Base eingesetzt wird, die nach Protonierung kein Wasser frei setzt.

2. Verfahren nach Anspruch 1, **dadurch gekennzeichnet, dass** R³ ein unsubstituierter Alkylrest mit 1 bis 4 C-Atomen ist.

3. Verfahren nach Anspruch 1, **dadurch gekennzeichnet, dass** der Carbonsäureester der allgemeinen Formel III einen Voll- oder Partialestern von Polyolen mit mindestens einer Säure R¹COOH darstellt.

4. Verfahren nach mindestens einem der Ansprüche 1 bis 4, **dadurch gekennzeichnet, dass** zwischen der Deprotonierung des Säureadditionsprodukts des Lysosphingolipids und biokatalytischer Herstellung ein Filtrationsschritt erfolgt.

5. Verfahren nach mindestens einem der Ansprüche 1 bis 4, **dadurch gekennzeichnet, dass** als Säureadditionsprodukt des Lysosphingolipids das Carbonsäurecarboxylat, Sulfat, Phosphat, Nitrat, Carbonat, Hydroxid oder Halogenid des Lysosphingolipids eingesetzt wird.

6. Verfahren nach mindestens einem der Ansprüche 1 oder 5, **dadurch gekennzeichnet, dass** als Base ein Alkalimetallalkoholat eingesetzt wird.

7. Verfahren nach mindestens einem der Ansprüche 1 bis 6, **dadurch gekennzeichnet, dass** das molare Verhältnis zwischen Säureadditionsprodukt des Lysosphingolipids und Base im Bereich zwischen 10 zu 1 bis 0,05 zu 1 beträgt.

8. Verfahren nach mindestens einem der Ansprüche 1 bis 7, **dadurch gekennzeichnet, dass** der Organismus des Reiches der Pilze ausgewählt ist aus der Gruppe der Gattungen *Aspergillus, Bipolaris, Candida, Fusarium, Geotrichum, Humicola, Microsporum, Mucor, Pichia, Thermomyces, Penicilium, Pichia, Rhizopus, Rhizomucor, Saccharomyces, Thermomyces, Trichosporon, Zygosaccharomyces.*

9. Verfahren nach mindestens einem der Ansprüche 1 bis 8, **dadurch gekennzeichnet, dass** die Biokatalysatormenge bezogen auf die Gesamtmasse an Edukten in einem Bereich zwischen 1% (w/w) und 100% (w/w) eingesetzt wird.

10. Verfahren nach mindestens einem der Ansprüche 1 bis 9, **dadurch gekennzeichnet, dass** der Biokatalysator zurückgewonnen wird.

11. Verfahren nach mindestens einem der Ansprüche 1 bis 10, **dadurch gekennzeichnet, dass** die Reaktanden zu Beginn der enzymatischen Umsetzung in einem molaren Verhältnis von Lysosphingolipid zu Carbonsäureester von 1:10 bis 10:1 vorliegen.

12. Verfahren nach mindestens einem der Ansprüche 1 bis 11, **dadurch gekennzeichnet, dass** die Reaktion im organischen Lösungsmittel durchgeführt wird.

13. Verfahren nach mindestens einem der Ansprüche 1 bis 12, **dadurch gekennzeichnet, dass** die Reaktion unter wasserfreien Bedingungen, definiert als Wassergehalt von maximal 0,1 M, nachgewiesen nach Karl Fischer, durchgeführt wird.

14. Verfahren nach mindestens einem der Ansprüche 1 bis 13, **dadurch gekennzeichnet, dass** die Reaktandenkonzentration zu Beginn der Reaktion im Bereich zwischen 0,01 M bis 3 M je Reaktand liegt.

15. Verfahren nach mindestens einem der Ansprüche 1 bis 14, **dadurch gekennzeichnet, dass** die Reaktionstemperatur im Bereich zwischen 20 °C und 130 °C liegt.

16. Verfahren nach mindestens einem der Ansprüche 1 bis 15, **dadurch gekennzeichnet, dass** die Reaktion unter einem Druck von kleiner 1 bar durchgeführt wird.

## Claims

1. Process for the biocatalytic preparation of sphingolipids of the general formula I 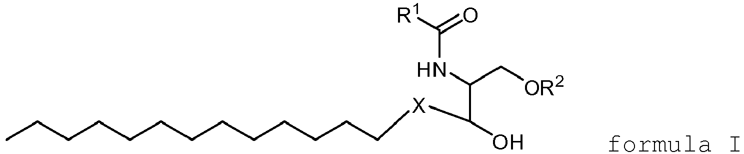 by
preparing a lysosphingolipid of the general formula II 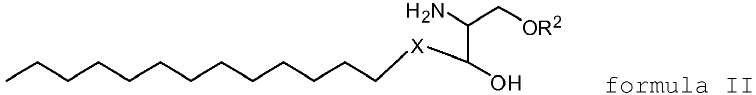 by deprotonation of an acid addition product, and reacting the lysosphingolipid with a carboxylic ester of the general formula III 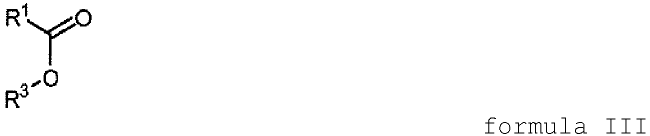
where R¹ represents a linear or branched alkyl chain which has 2 to 55 carbon atoms, optionally comprises one or more multiple bonds and/or aromatic or heteroaromatic rings, is optionally interrupted by oxygen atoms or ester or amide functionalities and is optionally substituted by at least one further group selected from alkyl, hydroxy, keto or amine groups,
R² represents H, phosphocholine, ethanolamine, serine or a sugar,
X represents CH=CH, CH₂-CH₂ or CH₂-HCOH,
R³ represents a branched or unbranched alkyl radical which has 1 to 12 carbon atoms and may be substituted with at least one radical -OR⁴,
where R⁴ independently is identical or nonidentical radicals selected from the group comprising
H
and
-C(O)R¹ where R¹ is as defined above,
**characterized in that** a biocatalyst used includes at least one carboxylic ester hydrolase of enzyme class E.C. 3.1.1 selected from the group comprising
carboxylic ester hydrolases of enzyme class E.C. 3.1.1 which can be isolated from an organism of the kingdom of fungi
and
carboxylic ester hydrolases of enzyme class E.C. 3.1.1 which are at least 80% homologous at the amino acid level with those which can be isolated from one organism of the kingdom of fungi, and
**in that** an organic or inorganic base which does not liberate any water after protonation is employed for the deprotonation.

2. Process according to Claim 1, **characterized in that** R³ is an unsubstituted alkyl radical having 1 to 4 carbon atoms.

3. Process according to Claim 1, **characterized in that** the carboxylic ester of the general formula III is a complete or partial ester of polyols with at least one acid R¹COOH.

4. Process according to at least one of Claims 1 to 4, **characterized in that** a filtration step takes place between the deprotonation of the acid addition product of the lysosphingolipid and biocatalytic preparation.

5. Process according to at least one of Claims 1 to 4, **characterized in that** the carboxylic acid carboxylate, sulphate, phosphate, nitrate, carbonate, hydroxide or halide of the lysosphingolipid is employed as acid addition product of the lysosphingolipid.

6. Process according to at least one of Claims 1 or 5, **characterized in that** an alkali metal alcoholate is employed as base.

7. Process according to at least one of Claims 1 to 6, **characterized in that** the molar ratio between acid addition product of the lysosphingolipid and base is in the range between 10:1 to 0.05:1.

8. Process according to at least one of Claims 1 to 7, **characterized in that** the organism of the kingdom of fungi is selected from the group of genera *Aspergillus, Bipolaris, Candida, Fusarium, Geotrichum, Humicola, Microsporum, Mucor, Pichia, Thermomyces, Penicillium, Pichia, Rhizopus, Rhizomucor, Saccharomyces, Thermomyces, Trichosporon, Zygosaccharomyces.*

9. Process according to at least one of Claims 1 to 8, **characterized in that** the amount of biocatalyst, based on the total mass of precursors, is employed in a range between 1% (w/w) and 100% (w/w).

10. Process according to at least one of Claims 1 to 9, **characterized in that** the biocatalyst is recovered.

11. Process according to at least one of Claims 1 to 10, **characterized in that** the reactants are present at the start of the enzymatic reaction in a molar ratio of lysosphingolipid to carboxylic ester of from 1:10 to 10:1.

12. Process according to at least one of Claims 1 to 11, **characterized in that** the reaction is carried out in the organic solvent.

13. Process according to at least one of Claims 1 to 12, **characterized in that** the reaction is carried out under anhydrous conditions, defined as a water content not exceeding 0.1 M detected by the Karl Fischer method.

14. Process according to at least one of Claims 1 to 13, **characterized in that** the reactant concentration at the start of the reaction is in the range between 0.01 M to 3 M for each reactant.

15. Process according to at least one of Claims 1 to 14, **characterized in that** the reaction temperature is in the range between 20°C and 130°C.

16. Process according to at least one of Claims 1 to 15, **characterized in that** the reaction is carried out under a pressure of less than 1 bar.

## Revendications

1. Procédé pour la préparation biocatalytique de sphingolipides de formule générale I 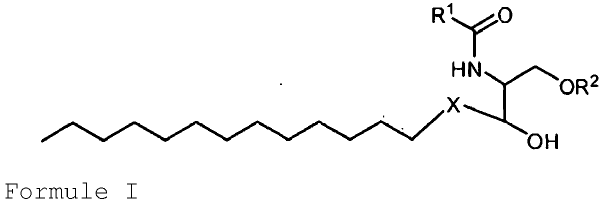 par préparation d'un lysosphingolipide de formule générale II, 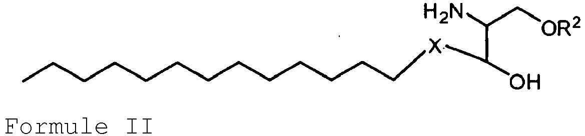 par déprotonation d'un produit d'addition d'acide et transformation du lysosphingolipide avec un ester d'acide carboxylique de formule générale III 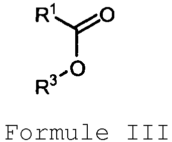 dans lesquelles
R¹ représente une chaîne alkyle comprenant 2 à 55 atomes de carbone, linéaire ou ramifiée, contenant le cas échéant un(e) ou plusieurs liaisons multiples et/ou cycles aromatiques ou hétéroaromatiques, le cas échéant interrompue par des atomes d'oxygène ou des fonctionnalités ester ou amide, le cas échéant substituée par au moins un autre groupe choisi parmi les groupes alkylhydroxy, céto ou amine,
R² représente H, phosphocholine, éthanolamine, sérine ou un sucre,
X représente CH=CH, CH₂-CH₂ ou CH₂-HCOH,
R³ représente un radical alkyle ramifié ou non ramifié comprenant 1 à 12 atomes de carbone, qui peut être substitué par au moins un radical -OR⁴,
où
R⁴ représente, indépendamment, des radicaux identiques ou différents du groupe comprenant H et -C(O)R¹, avec R¹ tel que défini ci-dessus
**caractérisé en ce qu'**on utilise un biocatalyseur qui présente au moins une carboxylester-hydrolase de la classe d'enzymes E.C. 3.1.1, choisie dans le groupe comprenant
les carboxylester-hydrolases de la classe d'enzymes E.C. 3.1.1, qui peuvent être isolées à partir d'un organisme du règne des champignons et
les carboxylester-hydrolases de la classe d'enzymes E.C. 3.1.1, qui sont homologues, sur le plan des acides aminés, à raison d'au moins 80% à celles qui peuvent être isolées à partir d'un organisme du règne des champignons, et **en ce qu'**on utilise, pour la déprotonation, une base organique ou inorganique, qui ne libère pas d'eau après la protonation.

2. Procédé selon la revendication 1, **caractérisé en ce que** R³ représente un radical alkyle non substitué comprenant 1 à 4 atomes de carbone.

3. Procédé selon la revendication 1, **caractérisé en ce que** l'ester d'acide carboxylique de formule générale III représente un ester complet ou partiel de polyols avec au moins un acide R¹COOH.

4. Procédé selon au moins l'une quelconque des revendications 1 à 4, **caractérisé en ce qu'**une étape de filtration a lieu entre la déprotonation du produit d'addition d'acide du lysosphingolipide et la préparation biocatalytique.

5. Procédé selon au moins l'une quelconque des revendications 1 à 4, **caractérisé en ce qu'**on utilise, comme produit d'addition d'acide du lysosphingolipide, le carboxylate d'acide carboxylique, le sulfate, le phosphate, le nitrate, le carbonate, l'hydroxyde ou l'halogénure du lysosphingolipide.

6. Procédé selon au moins l'une quelconque des revendications 1 ou 5, **caractérisé en ce qu'**on utilise, comme base, un alcoolate de métal alcalin.

7. Procédé selon au moins l'une quelconque des revendications 1 à 6, **caractérisé en ce que** le rapport molaire entre le produit d'addition d'acide du lysosphingolipide et la base se situe dans la plage entre 10:1 et 0,05:1.

8. Procédé selon au moins l'une quelconque des revendications 1 à 7, **caractérisé en ce que** l'organisme du règne des champignons est choisi dans le groupe des genres Aspergillus, Bipolaris, Candida, Fusarium, Geotrichum, Humicola, Microsporum, Mucor, Pichia, Thermomyces, Penicilium, Pichia, Rhizopus, Rhizomucor, Saccharomyces, Thermomyces, Trichosporon, Zygosaccharomyces.

9. Procédé selon au moins l'une quelconque des revendications 1 à 8, **caractérisé en ce que** la quantité de biocatalyseur, par rapport à la masse totale de produits de départ, est utilisée dans une plage entre 1% (P/P) et 100% (P/P).

10. Procédé selon au moins l'une quelconque des revendications 1 à 9, **caractérisé en ce que** le biocatalyseur est récupéré.

11. Procédé selon au moins l'une quelconque des revendications 1 à 10, **caractérisé en ce que** les réactifs, au début de la transformation enzymatique, se trouvent dans un rapport molaire de lysosphingolipide à ester d'acide carboxylique de 1:10 à 10:1.

12. Procédé selon au moins l'une quelconque des revendications 1 à 11, **caractérisé en ce que** la réaction est réalisée dans un solvant organique.

13. Procédé selon au moins l'une quelconque des revendications 1 à 12, **caractérisé en ce que** la réaction est réalisée dans des conditions anhydres, définies comme teneur en eau d'au maximum 0,1 M, prouvée selon Karl Fischer.

14. Procédé selon au moins l'une quelconque des revendications 1 à 13, **caractérisé en ce que** la concentration en réactifs au début de la réaction se situe dans la plage entre 0,01 M et 3 M par réactif.

15. Procédé selon au moins l'une quelconque des revendications 1 à 14, **caractérisé en ce que** la température de réaction se situe dans la plage entre 20°C et 130°C.

16. Procédé selon au moins l'une quelconque des revendications 1 à 15, **caractérisé en ce que** la réaction est réalisée à une pression inférieure à 1 bar.
